# NEW EUROPEAN PATENT SPECIFICATION

(11) **EP 1 349 456 B3**
(45) Date of publication and mention of the opposition decision: **14.10.2009**
(45) Mention of the grant of the patent: 28.12.2005
(21) Application number: 01998203.2
(22) Date of filing: 26.11.2001
(51) Int. Cl.: A01N 53/00, A01P 7/02

(54) **USE OF COMPOSITIONS FOR ENHANCED ACARICIDAL ACTIVITY**
VERWENDUNG VON ZUSAMMENSETZUNGEN ZUR VERBESSERUNG DER AKARIZIDEN WIRKSAMKEIT
UTILISATION DE COMPOSITIONS DESTINEES A PRODUIRE UNE ACTIVITE ACARICIDE AMELIOREE

(30) Priority: 30.11.2000 US 727117
(43) Date of publication of application: 08.10.2003
(73) Proprietor: Bayer HealthCare LLC, Tarrytown, New York 10591 (US)
(72) Inventor: ARTHER, Robert, G., Leawood, KS 66206 (US)
(74) Representative: von Renesse, Dorothea
(86) International application number: PCT/US2001/044084
(87) International publication number: WO 2002/043494

(56) References cited:
- EP-A- 0 387 663
- EP-A- 0 682 869
- EP-A1- 0 682 869
- WO-A-96/17520
- WO-A1-01//35739
- WO-A1-02//30200
- US-A- 5 661 164
- US-B1- 6 218 416
- DATABASE WPI Section Ch, Week 198827 Derwent Publications Ltd., London, GB; Class C02, AN 1988-188015 XP002198553 & JP 63 126805 A (NIHON TOKUSHU NOYAKU SEIZO KK), 30 May 1988 (1988-05-30)
- DATABASE CROPU [Online] R.ARTHER ET AL.: "Comparative evaluation of topically applied imidacloprid, permethrin, selamectin and fipronil for control of fleas and ticks on dogs" retrieved from STN-INTERNATIONAL, accession no. 2002-81364 CROPU XP002198549 & PROC.AM.ASSOC.VET.PARASITOL. (46 MEET., 38, 2001),
- DATABASE BIOSIS [Online] BIOSCIENCES INFORMATION SERVICE, PHILADELPHIA, PA, US; A.LIEBISCH ET AL.: "The efficacy of imidacloprid against flea infestation on dogs compared with three other topical preparations" retrieved from EPOQUE, accession no. PREV200000261492 XP002198550 & CANINE PRACTICE, vol. 25, no. 2, April 2000 (2000-04), pages 8-11,
- DATABASE MEDLINE [Online] M.FRANC ET AL.: "Activity of deltamethrin shampoo against Ctenocephalides felis and Rhipicephalus sanguineus in dogs" retrieved from EPOQUE, accession no. nlm10206106 XP002198551 & VETERINARY PARASITOLOGY, vol. 81, no. 4, 15 March 1999 (1999-03-15), pages 341-346,
- DATABASE BIOSIS [Online] BIOSCIENCES INFORMATION SERVICE, PHILADELPHIA, PA, US; W.J.GLADNEY ET AL.: "THE BROWN TICK LABORATORY TESTS OF ACARICIDES" retrieved from EPOQUE, accession no. PREV197764013791 XP002198552 & SOUTHWESTERN ENTOMOLIGIST, vol. 1, no. 4, 1976, pages 184-189,
- 'Products Power Plus, Company History, excerpt from homepage www.lab-brouwer.com.ar' PRODUCT LIST OF BROUWER S.A. ARGENTINA,
- 'Confirmation from SENASA that Marketing Authorization for the product POWER PLUS SPOT ON has been granted in Argentina to Brouwer S.A.' 05 March 1999,
- 'The Merck Index, 11th edition', 1989 article 'Permethrin', page 1138

## Description

### BACKGROUND OF THE INVENTION

Field of the Invention: The present invention relates to compositions for controlling acarids by means of a combination of pyrethroids and nicotinyl compounds. More specifically, the invention relates to compositions comprising a combination of pyrethroids and chloronicotinyl compounds, which produce enhanced activity against acarids, particularly ticks and mites on mammals, and on premises.

Brief Description of the Prior Art: Of particular interest here are compositions that are effective against insects such as fleas and acarids such as ticks and mites. Pyrethroids are known to be useful against acarids. Illustratively, U.S. Patent 5,236,954 discloses a liquid phase composition of a pyrethroid in concentrations greater than 50% w/w that may be used as basis for other pyrethroid-containing formulations in physical phases other than the liquid phase and methods of using the same as parasiticides. Also, nicotinyl compounds, particularty chloronicotinyl, are known to be effective against fleas. PCT application WO 93/24 002 discloses that certain 1-[N-(halo-3-pyeidylmethyl)]-N-methylamino-i-alkylamino-2-nitroethylene derivatives are suitable for systemic use against fleas in domestic animals. U.S. Patent 6,001,858, discloses the dermal application of chloronicotinyl compounds, which are particularly suitable for control of parasitic insects such as fleas, lice or files on animals.

It was, however, not known whether the addition of pyrethroids to nicotinyl compounds would enhance the activity of the pyrethroids without adversely affecting the activity of the latter against fleas. Surprisingly, the combination of the pyrethroids and nicotinyl compounds has been found to produce enhanced acaricidal activity as well as maintain continued excellent activity against fleas.

WO 96/17520 relates to parasiticidal formulations comprising a nicotinyl compound and optionally other active ingredients including natural or synthetic pyrethroids.

EP 0 387 663 discloses the combination of nitroimino imidazolines with pyrethroids.

JP 63-126805 discloses insecticidal compositions comprising imino-substituted heterocyclic compounds, i.a. imidacloprid, and a carboxylic acid ester, such as permethrin.

US 5,661,164 discloses a termite-controlling agent composition comprising a combination of imidacloprid and permethrin, a solvent and an emulsifer.

### SUMMARY OF THE INVENTION

In accordance with the foregoing, the present invention encompasses the use of a combination of a pyrethroid and a nicotinyl compound for the preparation of a product for the control of parasitic acarids on animals.

The composition is particularly suitable for dermal control of parasitic acarids and insects, particularly ticks, mites and fleas on mammals, as well, as premise control of fleas, ticks and mites and other susceptible insects. By the term " control" or "controlling" herein is meant rendering the insects and acarids innocuous, preferably by killing the insect and acarids to the extent that at least 80% die within days; and preferably within 2 days of application. In the preferred embodiment, the treated target is infested with insects and/or acarids. By the term combination is meant a regimen of applying the two active ingredients, either together or separately but concurrently.

In the presently preferred embodiment, the invention encompasses the use of a composition comprising a combination of permethrin and imidacloprid. It has been found that the combination of these active ingredients produces a synergistic effect of significantly enhancing onset of activity (control) against acarids such as ticks and mites, and longterm activity (control) against ticks and fleas. This is rather unexpected because imidacloprid or permethrin alone generally has limited activity against acarids such as ticks and mites, and permethrin alone, generally, has limited and short duration of activity against fleas. Surprisingly, imidacloprid in combination with permethrin has been found to significantly enhance the kill activity against these parasites, and thus provides excellent control. Moreover, in the use of the combination against fleas, imidacloprid activity has not been negatively affected by the permethrin.

The invention relates to the use of a combination of a pyrethroid and a nicotinyl compound for the preparation of a product for the control of parasitic acarids on animals comprising a formulation for dermal application to mammals, wherein the pyrethroid is present in concentrations from 40% to 60% (w/w) , with the exception of the use for the control of ticks when the product contains deltamethrin and acetamiprid.

The invention further relates to the use of a combination of a pyrethroid and a nicotinyl compound for the preparation of a product for the control of parasitic acarids on animals, wherein the pyrethroid and the nicotinyl compound are contained in two separate formulations for concurrent application.

### DETAILED DESCRIPTION OF THE INVENTION

As set forth above, the invention relates to the use of a composition comprising a combination of pyrethroids and chloronicotinyl compounds in effective concentrations to provide enhanced acaricidal activity without producing a detrimental effect on the activity of nicotinyl compounds on fleas. Pyrethroid insecticides including such compounds as permethrin, cyfluthrin, flumethrin and fenvalerate are more stable synthetic analogues of the naturally occurring pyrethrins. Pyrethroids bind to the membrane receptors along the nerve axon, causing prolonged opening of the sodium channels, resulting in prolonged depolarization, repetitive nerve firing and synaptic disturbances leading to hyperexcitatory symptons. Nicotinyl compounds have a distinct mode of action with biological activities that are different anatomically and physiologically from the pyrethoids. They bind to the nicotinergic receptors in the post-synaptic nerve region, which prevents acetylcholine chemical transmitter of signals between nerves from binding and transmitting signals. Reportedly, the chloronicotinyl compounds are more specific than pyrethroids for the binding sites on insect nerves than acarids or vertebrtates.

Without being bound to any particular theory of the invention, It is believed that the nicotinyl compounds do not bind to sufficient number of receptor sites on acarid post-synaptic nerve locations to provide activity. The chloronicotinyl compounds are, therefore, ineffective or only marginally active against ticks and mites.

Surprisingly, the combination of a pyrethroid and a chloronicotinyl insecticide provides enhanced activity against ticks and mites, while maintaining the activity of chloronicotinyl compounds against fleas. The enhanced activity is most notable when the two compounds are first applied producing a faster kill of acarids than permethrin alone and then again at the end of the effective treatment duration when the effects of the pyrethroid alone begins to decline.

Illustrative but non-limiting examples of pyrethroids are permethrin, phenthrin, cypermethrin, cyhalothrin, lambda cyhalothrin, cyfluthrin, cyphenothrin, tralomethrin, tralocythrin, deltamethrin, slubalinate, fluvatinate, flumethrin and fenvalerate. Preferred herein is permethrin, [(3-phenoxy-phenyl)methyl-3-92,2-dichlorovinyl)-2,2-dimethlycyclopropanecarboxylate].

Chloronicotinyl compounds are known, for example, from European Offenlegungsschriften (European Published Applications) Nos. 580 553, 464 830, 428 941, 425 978, 386 565, 383 091, 375 907, 364 844, 315 826, 259 738, 254 859, 235 725, 212 600, 192 060, 163 855, 154 178, 136 636, 303 570, 302 833, 306 696, 189 972, 455 000, 135 956, 471 372, 302 389; German Offen-legungsschriften (German Published Specifications) Nos. 3 639 877, 3 712 307; Japanese Offenlegungs-schriften (Japanese Published Applications) Nos. 03 220 176, 02 207 083, 63 307 857, 63 287 764; 03 246 283, 04 9371, 03 279 359, 03 255 072, U.S. Patents 5,034,524,4,948,798,4,918,086,5,039,686 and 5,034,404; PCT Applications Nos. WO 91/17 659, 91/4965; French Application No. 2 611 114; and Brazilian Application No. 88 03 621.

These compounds can be advantageously represented by the general formula (I) 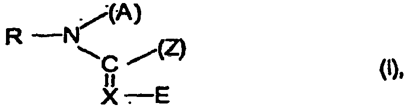 in which
- R: represents, hydrogen, optionally, substituted radicals from acyl, alkyl, aryl, aralkyl, heteroaryl or heteroarylalkyl;
- A: represents a monofunctional group from hydrogen, acyl, alkyl, aryl, of represents a bifunctional group which is in to the radical Z;
- E: represents an electron-withdrawing radical;
- X: represents the radicals -CH= or =N-, it being possible for the radical -CH= instead of an H atom to be linked to the radical Z;
- Z: represents a monofunctional group from alkyl, -O-R, -S-R, 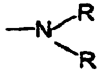
or represents a bifunctional group which is linked to the radical A or to the radical X.

Particularly preferred compounds of the formula (I) are those in which the radicals have the following meaning:
- R: represents hydrogen and represents optionally substituted radicals from acyl, alkyl, aryl, aralkyl, heteroaryl, heteroarylalkyl.

Acyl radicals which may be mentioned are formyl, alkylcarbonyl, arylcarbonyl, alkylsulfonyl, arylsulfonyl, (alkyl)-(aryl)-phosphoryl, which may in turn be substituted.

As alkyl there may be mentioned C₁₋₁₀-alkyl, especially C₁₋₄-alkyl, specifically methyl, ethyl, i-propyl, sec- or t-butyl, which may in turn be substituted.

As aryl there may be mentioned phenyl or naphthyl, especially phenyl.

As aralkyl there may be mentioned phenylmethyl or phenethyl.

As heteroaryl there may be mentioned heteroaryl having up to 10 ring atoms and N, O or S, especially N, as hetero atoms. Specifically there may be mentioned thienyl, furyl, thiazolyl, imidazolyl, pyridyl and benzothiazolyl.

As heteroarylalkyl there may be mentioned heteroarylmethyl or heteroarylethyl having up to 6 ring atoms and N, O or S, especially N, as hetero atoms.

Substituents which may be listed by way of example and preference are:
alkyl having preferably 1 to 4, in particular 1 or 2 carbon atoms, such as methyl, ethyl, n- and i-propyl and n-, i- and t-butyl; alkoxy having preferably 1 to 4, in particular 1 or carbon atoms, such as methoxy, ethoxy, n- and i-propyloxy and n-, i- and t-butyloxy; alkylthio having preferably 1 to 4, in particular 1 or 2 carbon atoms, such as methylthio, ethylthio, n- and i-propylthio and n-, i- and t-butylthio; halogenoalkyl having preferably 1 to 4, in particular 1 or 2 carbon atoms and preferably 1 to 5, in particular 1 to 3 halogen atoms, the halogen atoms being identical or different and being preferably fluorine, chlorine or bromine, especially fluorine, such as trifluoromethyl; hydroxyl; halogen, preferably fluorine, chlorine, bromine and iodine, especially fluorine, chlorine and bromine; cyano; nitro; amino; monoalkyl- and dialkylamino having preferably 1 to 4, in particular 1 or 2 carbon atoms per alkyl group, such as methylamino, methyl-ethyl-amino, n- and i-propylamino and methyl-n-butylamino; carboxyl; carbalkoxy having preferably 2 to 4, in particular 2 or 3 carbon atoms, such as carbomethoxy and carboethoxy; sulpho (-SO₃H); alkylsulfonyl having preferably 1 to 4, in particular 1 or 2 carbon atoms, such as methylsulfonyl and ethylsulfonyl; arylsulfonyl having preferably 6 or 10 aryl carbon atoms, such as phenylsulfonyl, and also heteroarylamino and heteroaryla-Ikylamino such as chloropyridylamino and chloropyridylmethylamino.

A particularly preferably represents hydrogen and represents optionally substituted radicals from acyl, alkyl or aryl, which preferably have the meanings given for R. A additionally represents a bifunctional group. There may be mentioned optionally substituted alkylene having 1-4, in particular 1-2 C atoms, substituents which may be mentioned being the substituents listed earlier above, and it being possible for the alkylene groups to be interrupted by hetero atoms from the group consisting of N, O or S.

A and Z may, together with the atoms to which they are attached, form a saturated or unsaturated heterocyclic ring. The heterocyclic ring can contain a further 1 or 2 identical or different hetero atoms and/or hetero groups. Hetero atoms are preferably oxygen, sulfur or nitrogen, and hetero groups are preferably N-alkyl, where the alkyl in the N-alkyl group preferably contains 1 to 4, in particular 1 or 2 carbon atoms. As alkyl there may be mentioned methyl, ethyl, n-and i-propyl and n-, i- and t-butyl. The heterocyclic ring contains 5 to 7, preferably 5 or 6 ring members.

Examples of the heterocyclic ring which may be mentioned are imidazolidine, pyrrolidine, piperidine, piperazine, hexamethyleneimine, hexahydro-1,3,5-triazine, hexahydrooxodiazine and morpholine, each of which may optionally be substituted, preferably by methyl.
- E: represents an electron-withdrawing radical, in which context particular mention may be made of NO₂, CN and halogenoalkyl- carbonyl such as 1,5-halogeno-C₁₋₄-carbonyl, especially COCF₃.
- X: represents -CH= or -N=
- Z: represents optionally substituted radicals alkyl, -OR, -SR or -NRR, where R and the substituents preferably have the meaning given above.
- Z: can form, apart from the above-mentioned ring, and together with the atom to which it is attached and with the radical =C- instead of X, a saturated or unsaturated heterocyclic ring. The heterocyclic ring can contain a further 1 or 2 identical or different hetero atoms and/or groups. The hetero atoms are preferably oxygen, sulfur or nitrogen, and the hetero groups are preferably N- alkyl, in which case the alkyl or N-alkyl group preferably contains 1 to 4, in particular 1 or 2 carbon atoms. As alkyl there may be mentioned methyl, ethyl, n- and i-propyl and n-, i- and t-butyl. The heterocyclic ring contains 5 to 7, preferably 5 or 6 ring members.

Examples of the heterocyclic ring which may be mentioned are pyrrolidine, piperidine, piperazine, hexamethyleneimine, morpholine and N-methylpiperazine.

Particularly preferred are compounds of the general formulae (II) and (III): 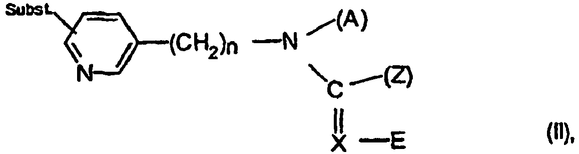 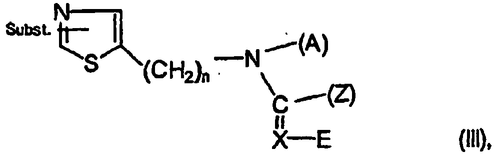 in which
n represents 1 or 2,
Subst. represents one of the above-listed substituents, especially halogen, very particularly chlorine, A, Z, X and E have the meanings given above,

Specifically, the following compounds may be mentioned:

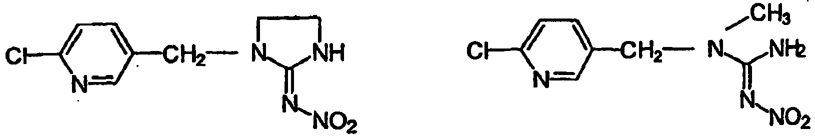

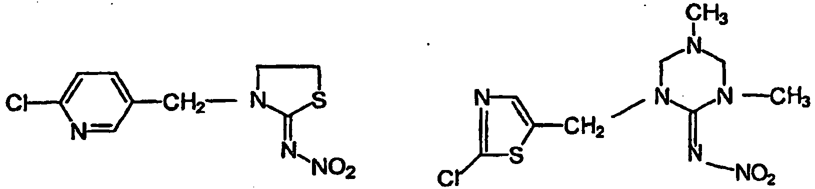

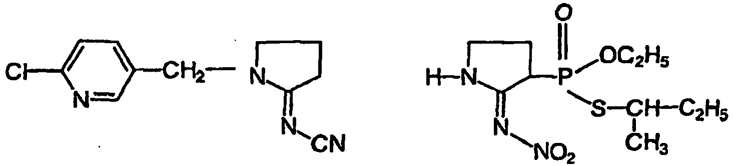

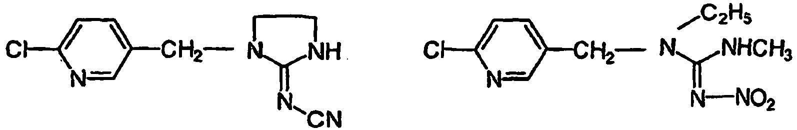

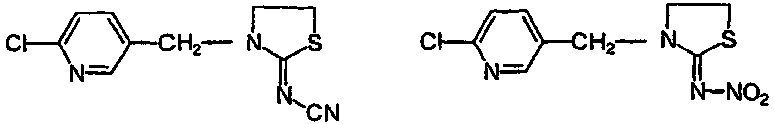

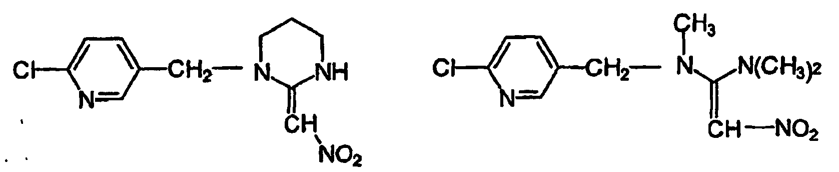

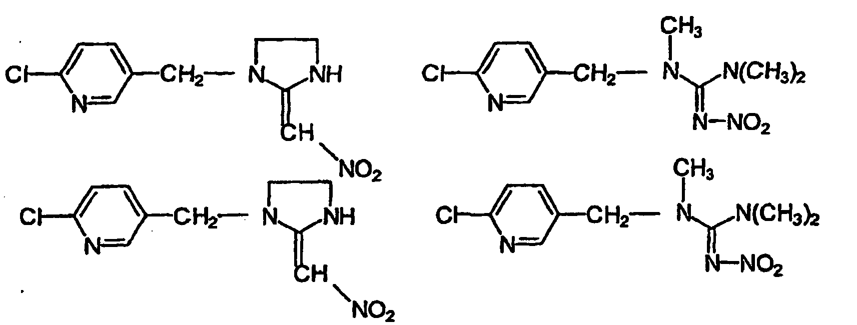

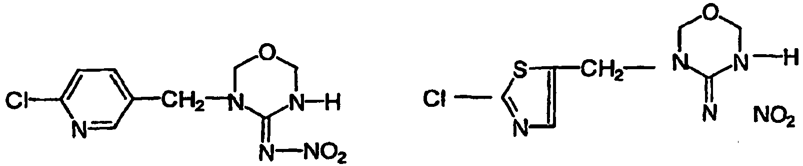

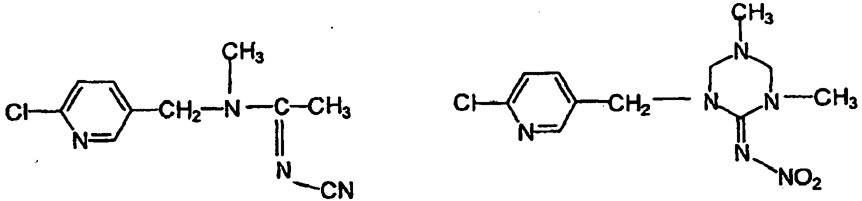

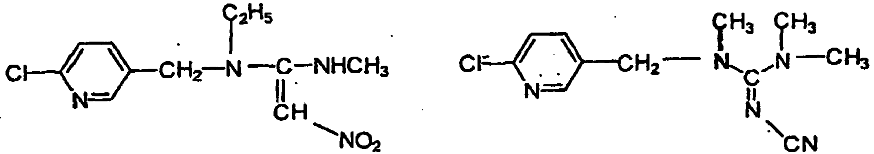

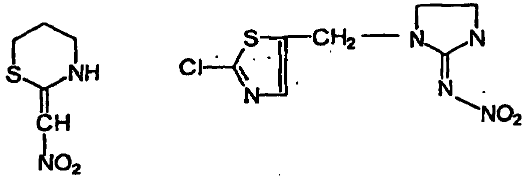

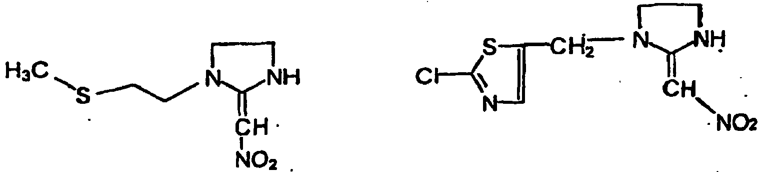

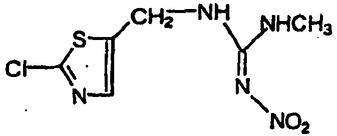

In the method of preparing a composition which is useful according to the invention, the active ingredients can be combined in any convenient manner such as in an aqueous solution, suspension or emulsion or solid matrices such as ear tags or collars. Preferably, both active ingredients are soluble in one or more solvents used in the formulation. The active ingredients may be combined by mixing with extenders such as liquid solvents, pressurized liquified gases and/or solid carriers, optionally with the use of surfactants.

The concentration of the active ingredients in the composition or formulation is such as is effective to control the parasitic insects or acarids. The particular concentration would depend on the form of the formulation and the method of application. Typically, the pyrethroid can be present in concentrations of from 0.1 % to 60% w/w depending on the use (premise or dermal application on mammals) and preferably from 40% to 60% (w/w) for dermal application to mammals. The nicotinyl compounds can be present in concentrations of 0.001 % to 60% (w/w) depending on the use (premise or dermal application on mammals) and preferably from 0.1 % to 25% (w/w) for dermal application on mammals. Most preferably, the composition comprises at least 40% (w/w) permethrin and 8-10% (w/w) imidacloprid. Preparations which are diluted before use contain the active substance in concentrations of from 0.1% (w/w) to 90% (w/w). For dermal application to animals, the formulation preferably contains from 0.1% (w/w) to 25% (w/w), preferably from 5% (w/w) to 20% (w/w). Given the teachings herein, it will be within the purview of the skilled artisan to select the type and concentration of pyrethroids that are not toxic to mammals, particularly cats.

Solvents useful herein can be selected from the group consisting of but not limited to water, oils, pyrrolidones, alcohols and cyclic carbonates; optionally with co-solvents from similar groups. Preferred oils include light mineral oil and vegetable oils. Preferred pyrrolidones include but are not limited to N-methyl pyrrolidone. Preferred alcohols include but are not limited to aromatic or aliphatic alcohols such as glycols, benzyl alcohol, isopropanol, ethanol, diethylene glycol, propylene glycol, 2-octyl-1-dodecanol and tetrahydrofurfuryl alcohol. They are present in a concentration of at least 0.01 to 95% by weight, preferably from 1 to 30% by weight, particularly preferably from 1 to 20% by weight. Preferred cyclic carbonates are ethylene carbonate and propylene carbonate. Particular preferred is propylene carbonate which can be present in a concentration of from 2.5 to 99.9999% by weight, preferably from 7.5 to 90% by weight, particularly preferably from 10 to 90% by weight.

Suitable further auxiliaries are: preservatives such as benzyl alcohol (not required if already present as solvent), trichlorobutanol, p-hydroxybenzoic esters, n-butanol, piperonyl butoxide and water as solubility enhancer. They are present in a concentration of from 0 to 15% by weight, preferably from 2.5 to 12.5% by weight, particularly from 2.5 to 10.0% by weight. The sum of active compounds, solvents and auxiliaries has to be 100% by weight.

Thickeners are, for example, inorganic thickeners such as bentonites, colloidal silicic acid, aluminum monostearate, organic thickeners such as cellulose derivatives, polyvinyl alcohols, polyvinylpyrrolidones and copolymers thereof, acrylates and methacrylates.

Colorants useful herein are those approved for use in drugs which may be dissolved or suspended.

Spreading agents include but are not limited to oils such as di-2-ethylhexyl adipate, isopropyl myristate, dipropylene glycol pelargonate, cyclic and acyclic silicone oils such as dimeticones and also co- and terpolymers thereof with ethylene oxide, propylene oxide and formalin, fatty acid esters, triglycerides and fatty alcohols.

Antioxidants are, for example, sulfites ormetabisulfites such as potassium metabisulfite, ascorbic acid, butylated hydroxytoluene, butylated hydroxyanisole, tocopherol. Light stabilizers are, for example, substances from the class of the benzophenones or Novantisol acid. Adhesives are, for example, polymeric thickeners, for example, cellulose derivatives, starch derivatives, polyacrylates, naturally occurring polymers such as alginates and gelatin.

Auxiliaries are also emulsifiers such as nonionic surfactants, for example polyoxyethylated castor oil, polyoxyethylated sorbitan monooleate, sorbitan monostearate, glycerol monostearate, polyoxyethyl stearate, alkylphenol polyglycol ethers; ampholytic surfactants such as disodium N-lauryl-β-iminodipropionate or lecithin; anionic surfactants such as sodium lauryl sulfate, fatty alcohol ether sulfates, mono/dialkylpolyglycol ether orthophosphoric ester monoethanolamine salt; and cationic surfactants such as cetyltrimethylammonium, chloride.

While being of low toxicity to warm-blooded species, the formulations according to the invention are suitable for the control of parasitic insects which are encountered on premises, and animals including dogs, cats, horses, cattle, swine, sheep and humans. They are active against all or individual stages of development of the pests and against resistant and normally sensitive species of the pests.

In the practice of the invention, the composition can be applied in any convenient manner. In dermal applications, for example, the composition can be applied by dropping a small but effective volume at a spot on the animal. In the present embodiment of the invention, synergistic results are obtained when the active ingredients are applied concurrently as separate formulations. A combination of the pyrethroids and nicotinyl compound in a single formulation is preferred.

The combination is particularly effective against Siphoneptera (fleas), and Acarina (ticks and mites). Surprisingly, the combination has been found to be particularly effective against the species of ticks on dogs, *Demacentor variabilis* and *Rhipicephalus sanguineus.* The results are unexpected because the agonist or antagonists of acetycloline receptors of insects such as imidacloprid have no appreciable activity against acarids such as ticks and mites; yet the combination thereof with permethrin results in a substantially enhanced activity against these parasites. Additionally, the exceptional activity of chloronicotinyl compounds against fleas is not reduced.

The composition according to the invention may additionally comprise other active ingredients such as insect growth regulants (pyriproxifen, methoprene, which do not interfere with the preparation or efficacy of the combination.

Active compounds which can be used for the purposes of the invention include imidacloprid, AKD 1022 and Ti 435. AKD 1022 is a chloronicotinyl derivative of the formula 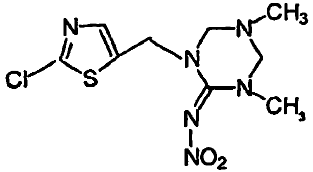 Ti 435 is a chloronicotinyl derivative of the formula

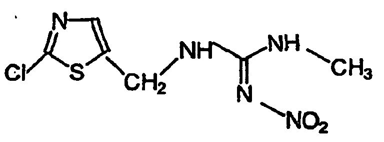

In the examples which follow, the active compounds employed are [(3-phenoxyphenyl)methyl-3-92,2-dichlorovinyl)-2,2-dimethlycyclopropanecarboxylate] having the common name permethrin and 1-[(6-chloro-3-pyridinyl)methyl]-N-nitro-2-imidazolidinimine having the common name imidacloprid.

The invention is further illustrated but is not intended to be limited by the following examples in which all parts and percentages are by weight unless otherwise specified.

### EXAMPLES

### Example 1

The purpose of this study was to was to determine comparative flea and tick control over a 30 day interval of a combination application of a pyrethroid and a chloronicotinyl insecticide applied dermally to dogs. This combination was compared with permethrin alone, imidacloprid alone, fipronil and selamectin. The latter two compounds are present in products that currently carry claims for both tick and flea control

Thirty-six dogs were divided into six groups of 6 dogs per group. Each dog received a single topically-applied treatment of the either "Kiltix", a product available from Bayer Corporation containing 45% w/w permethrin, Advantage®, a product available from Bayer Corporation containing 9.1 % w/w imidacloprid, a combination of Kiltix and Advantage containing 45% w/w permethrin + 9.1 %w/w imidacloprid, Top Spot®, a product available from Merial containing 9.7% fipronil or Revolution®, a product available from Pfizer Inc. containing 12% w/v selamectin in accordance with the appropriate dose and label instructionsforthe various product applications. Control dogs remained untreated. All products were provided in the commercial unit dose applicator tubes.

The dogs were bathed with a mild non-medicated shampoo and thoroughly combed to remove any existing fleas orticks 7 to 14 days prior to treatment. The dogs were infested with 100 unfed adult ticks (50 *Dermacentor variabilis* and 50 *Rhipiciphalus sanguineus*) and 100 unfed adult fleas on Day -3. Live fleas and ticks were counted on Day -1. The dogs were ranked according to total pretreatment live tick counts from highest to lowest. The 36 dogs with the highest counts were selected for the study. Each consecutive group of 6 dogs comprised one block. Treatment was randomly assigned within each block of dogs.

Each dog was examined visually for fleas and ticks on Days 1, 7, 14, 21, and 28 following treatment. The hair was parted with the thumbs and fingers to count fleas and ticks. Live tick counts were recorded by species. Live ticks only were counted visually on Days 2, 8, 15, 22, and 29. The dogs were combed on Days 3, 9, 16, 23, and 30. All remaining live fleas and ticks were counted and removed.

The dose for the various compounds is provided in Table 1.

**Table 1 Dose of Compounds Dermally Applied to Dogs**

| Group | Treatment | Dose | Application |
|---|---|---|---|
| 1 | 45% Permethrin | <33 lbs = 1.5 mL | <33 lbs: 1.5 mL of solution on the back between the shoulder blades |
| | | >33 lbs = 2x1.5 mL | > 33 lbs: 1.5 mL between the shoulder blades +1.5 mL on the rump at the base of the tall |
| 2 | 9.1% Imidacloprid | <101b=0.4mL | On the back to one spot between the shoulder blades Apply evenly to 3-4 spots on the back between shoulderto base of tall |
| | | 11-20 lb = 1.0 mL | |
| | | 21 - 55 lb = 2.5 mL | |
| | | >55 lb = 4.0 mL | |
| 3 | 45% Permethrin + 9.1 % Imidacloprid | Same as above for both products | Apply according to above directions but do not apply both products to the same spot |
| 4 | 9.7% fipronil | <22 lbs = 0.67 mL | Apply contents of tube on the skin at one spot between the shoulder blades |
| | | 23-44 lbs = 1.3 mL | |
| | | 45-48 lbs = 2.68 mL | |
| 5 | 12% Selamectin (120 mg/mL) | 10.1-20 lb = 0.5 mL | Apply contents of tube on the skin at one spot between the shoulder blades |
| | | 20.1-40 lb= 1.0 mL | |
| | | 40.1-85 lb = 2.0 mL | |
| 6 | Control | No Treatment | |

The results of this study are shown in Table 2, 3 and 4.

**Table 2**

| COMPARATIVE EFFICACY | | | | | |
|---|---|---|---|---|---|
| *D. VARIABIUS* | | | | | |
| **PERCENT CONTROL** | | | | | |
| Study Day | Imidacloprid | Permethrin | Imidacloprid+Permethrin | Fipronil | Selamectin |
| 1 | -12.0 | 36.2 | 64.2 | *92.9 | 26.2 |
| 2 | 16.9 | 53.9 | 81,9 | *100 | 46.7 |
| 3 | 30.9 | 75.3 | 96.4 | 100 | 70.8 |
| 7 | 32.5 | 95.2 | 97.0 | 100 | 23.1 |
| 8 | 35.3 | 96.1 | 98.4 | 100 | 61.2 |
| 9 | 39.4 | 97.1 | 98.6 | 100 | 83.2 |
| 14 | 50.3 | 91.5 | 97.4 | 98.7 | 16.6 |
| 15 | 66.4 | 92.9 | 99.2 | 100 | 32.7 |
| 16 | 68.4 | 96.8 | 99.2 | 100 | 46.1 |
| 21 | 50.2 | 90.8 | 87.7 | 92.7 | 2.7 |
| 22 | 40.1 | 85.1 | 94.5 | 98.7 | -0.6 |
| 23 | 50.2 | 89.3 | 97.7 | 100 | 24.7 |
| 28 | 38.8 | 79.3 | *91.8 | 69.7 | 0.1 |
| *Flpronil significantly different than Imidacloprid + Permethrin | | | | | |
| "Imidacloprid + Permethrin significantly different than Fipronil | | | | | |

**Table 3**

| COMPARATIVE EFFICACY | | | | | |
|---|---|---|---|---|---|
| R. SANGUINEUS | | | | | |
| **PERCENT CONTROL** | | | | | |
| Study Day | Imidacloprid | Permethrin | Imidacloprid + Permethrin | Fipronil | Selamectin |
| 1 | 15.5 | 72.7 | 76.8 | 96.3 | -13.1 |
| 2 | 42.4 | 75.0 | 85.9 | 100 | 48.5 |
| 3 | 35.9 | 85.0 | 91.8 | 100 | 87.4 |
| 7 | 67.2 | 99.4 | 98.9 | 100 | 83.9 |
| 8 | 72.0 | 100 | 100 | 100 | 83.6 |
| 9 | 66.6 | 99.0 | 100 | 100 | 95.6 |
| 14 | 53.5 | 95.2 | 95:2 | 99.4 | 21.5 |
| 15 | 58.2 | 98.9 | 98.2 | 100 | 46.0 |
| 16 | 54.0 | 99.4 | 98.4 | 99.4 | 70.9 |
| 21 | 41.5 | 89.4 | 87.0 | 86.0 | -7.0 |
| 22 | 18.9 | 91.7 | 91.8 | 100 | -2.2 |
| 23 | -5.3 | 91.5 | 99.0 | 100 | 8.2 |
| 28 | 39.1 | 68.6 | 84.6 | 65.3 | -16.0 |

**TABLE 4**

| COMPARATIVE EFFICACY | | | | | |
|---|---|---|---|---|---|
| **FLEAS (*Siphoneptera*)** | | | | | |
| ***PERCENT CONTROL*** | | | | | |
| Day | Permethrin | Imidacioprid | Imidacloprid + Permethrin | Fipronil | Selameclin |
| -1 | 1.5 | 5.4 | 27.7 | 22.4 | 15.1 |
| 1 | 89.8 | 100 | 100 | 100 | 87.3 |
| 3 | 93.9 | 100 | 100 | 100 | 100 |
| 7 | 79.4 | 100 | 100 | 100 | 100 |
| 9 | 87.8 | 100 | 100 | 100 | 100 |
| 14 | 71.9 | 100 | 100 | 100 | 99.7 |
| 16 | 65.1 | 100 | 100 | 100 | 100 |
| 21 | 52.9 | 100 | 99.8 | 100 | 99.8 |
| 23 | 41.9 | 99.6 | 100 | 100 | 100 |
| 28 | 43.9 | 98.6 | 98.4 | 100 | 86 |
| 30 | 7.7 | 99.4 | 98.7 | 100 | 98 |

The following significant conclusions can be drawn from this study.
1. The combination of permethrin and imidacloprid produced a faster kill of both species of ticks (*D. variabilis* and *R. sanguineus*) than either permethrin or imidacloprid alone. The combination provided 82 to 86% killing of ticks by day 2 post application and approximately 100% killing of both species of ticks by day 3 post application. Permethrin alone required 7 days to approach a 100% killing of ticks. Selamectin required 9 days to reach only an 83% killing of *D. variabilis,* and then this compound lost its activity. Selamectin produced earlier killing of R. sanguineus (87% by day 3), however, the tick killing of selamectin decreased rapidly and was negligent by day 16 post application. Fipronil produced an early kill, similar to that of the combination of permethrin and imidacloprid.
2. The length of time that significant tick control occurred with the combination of permethrin and imidacloprid was significantly longer than that of permethrin alone, imidacloprid alone, selamectin or fipronil. The data indicate that the combination of permethrin and imidacloprid controlled 85 to 92 % of both species of ticks by 28 days post application.
3. The killing of fleas on dogs remained unaffected by the presence of permethrin in the formulation. Table4 indicates that permethrin alone had some killing effect on fleas from day 1 through day 21 whereas imidacloprid killed essentially all of the fleas from day 1 through day 30. The combination of permethrin and imidacloprid demonstrated an equally effective killing of fleas from day 1 through day 30. Selamectin was not as effective as either imidacloprid or the combination of imidacloprid and permethrin. The latter compound required 3 days to demonstrate a significant killing of fleas and then this killing effect appeared to fall by 28 days post application. Fipronil demonstrated a rate of flea kill equal to that of imidacloprid or the combination of imidacloprid and permethrin.
4. The rapid onset of killing of both fleas and ticks by the combination of permethrin and imidacloprid indicates that there was effective spreading of both active ingredients.
5. The length of time that the combination remained active against both species of ticks and fleas indicates that there is adequate distribution of the active ingredients into the skin of the animals.

The foregoing shows that a combination of a pyrethroid and a chloronicotinyl compound produces a synergistic effect against killing ticks and remains effective against killing of fleas. The killing effects on ticks began earlier and lasted longer with the combination than with either the permethrin or imidacloprid alone.

### Example 2

The above study incorporated an evaluation of safety of the various compounds. This was determined by examination of the skin at the site(s) of application and the behaviour of the dogs post application. None of the formulations produced an irritation at the site of application and none of the dogs demonstrated discomfort post application. Therefore, it was determined that the combination of permethrin and imidacloprid was safe, non-irritating and effective against ticks and fleas. It would be expected to be effective against mites as they have a response similar to ticks.

## Claims

1. Use of a combination of a pyrethroid and a nicotinyl compound for the preparation of a product for the control of parasitic acarids on animals comprising a formulation for dermal application to mammals, wherein the pyrethroid is present in concentrations from 40% to 60% (w/w)
with the exception of
the use for the control of ticks when the product contains deltamethrin and acetamiprid.

2. Use according to claim 1, wherein the pyrethroid is permethrin.

3. Use according to claim 1 or 2, wherein the nicotinyl compound is imidacloprid.

4. Use of a combination of a pyrethroid and a nicotinyl compound for the preparation of a product for the control of parasitic acarids on animals, wherein the pyrethroid and the nicotinyl compound are contained in two separate formulations for concurrent application.

## Patentansprüche

1. Verwendung einer Kombination aus einer Pyrethroid- und einer Nicotinylverbindung zur Herstellung eines Produkts zur Bekämpfung von parasitären Acarl bei Tieren, enthaltend eine Zusammensetzung zur dermalen Applikation bei Säugetieren, worin das Pyrethroid in einer Konzentration von 40 bis 60 Gew.-% vorliegt.
mit Ausnahme
der Verwendung zur Bekämpfung von Zecken, wenn das Produkt Deltamethrin und Acetamiprid enthält.

2. Verwendung nach Aspruch 1, worin das Pyrethroid Permethrin ist.

3. Verwendung nach den Ansprüchen 1 oder 2, worin die Nicotinylverbindung Imidacloprid ist.

4. Verwendung einer Kombination aus einer Pyrethrold- und einer Nicotinylverbindung zur Herstellung eines Produkts zur Bekämpfung von parasitären Acarl bei Tieren, worin die Pyrethroid- und die Nicotinylverbindung in zwei getrennten Formulierungen zur gleichzeitigen Anwendung enthalten sind.

## Revendications

1. Utilisation d'une combinaison d'un composé pyréthroïde et d'un composé nicotinyle pour la préparation d'un produit pour le contrôle des acariens parasites sur les animaux, comprenant une formulation pour l'application cutanée sur les mammifères, dans laquelle le composé pyréthroïde est présent à des concentrations de 40% à 60% en poids,
à l'exception de
l'utilisation pour le contrôle des tiques quand le produit contient de la deltaméthrine et de l'acétamiprid.

2. Utilisation selon la revendication 1, dans laquelle le composé pyréthroïde est la perméthrine.

3. Utilisation selon la revendication 1 ou 2, dans laquelle le composé nicotinyle est l'imidacloprid.

4. Utilisation d'une combinaison d'un composé pyréthroïde et d'un composé nicotinyle pour la préparation d'un produit pour le contrôle des acariens parasites sur les animaux, dans laquelle le composé pyréthroïde et le composé nicotinyle sont contenus dans deux formulations séparées pour l'application simultanée
